# EUROPÄISCHE PATENTSCHRIFT

(11) **EP 2 282 787 B1**
(45) Veröffentlichungstag und Bekanntmachung des Hinweises auf die Patenterteilung: **27.11.2013**
(21) Anmeldenummer: 09761313.7
(22) Anmeldetag: 29.05.2009
(51) Int. Cl.: A61M 1/00

(54) **VERFAHREN UND VORRICHTUNG ZUM TRENNEN VON GEWEBEZELLEN AUS EINER FLÜSSIGKEIT**
METHOD AND DEVICE FOR ISOLATING TISSUE CELLS FROM A LIQUID
PROCÉDÉ ET DISPOSITIF PERMETTANT D'ISOLER DES CELLULES TISSULAIRES CONTENUES DANS UN LIQUIDE

(30) Priorität: 10.06.2008 DE 102008027486
(43) Veröffentlichungstag der Anmeldung: 16.02.2011
(73) Patentinhaber: Human Med AG, 19061 Schwerin (DE)
(72) Erfinder: KENSY, Arnd, 14552 Michendorf - OT Wilhelmshorst (DE); WINKLER, Konrad-Wenzel, 19417 Warin (DE); UEBERREITER, Klaus, 16547 Birkenwerder (DE)
(74) Vertreter: Gulde Hengelhaupt Ziebig & Schneider
(86) Internationale Anmeldenummer: PCT/DE2009/000775
(87) Internationale Veröffentlichungsnummer: WO 2009/149691

(56) Entgegenhaltungen:
- EP-A1- 1 234 589
- US-A- 5 720 299
- US-A- 6 071 095
- US-A1- 2003 042 187
- US-A1- 2005 139 532

## Beschreibung

Die Erfindung bezieht sich auf ein Verfahren zum Trennen von Gewebezellen aus einer Flüssigkeit, bei dem ein Gemisch aus den Gewebezellen und der Flüssigkeit in einen Vakuumstrom eingebunden und in einem Gewebesammelbehälter wieder vom Vakuumstrom getrennt wird, wobei die Gewebezellen aus dem Gemisch durch die Schwerkraft ausgefiltert und die verbleibende Flüssigkeit wieder in den Vakuumstrom eingebunden wird.
Die Erfindung bezieht sich weiterhin auf eine Vorrichtung zum Trennen von Gewebezellen aus einer Flüssigkeit, bestehend aus einem unter Vakuum stehenden Gewebezellensammler mit einer Filtereinheit, die den Sammelbehälter in einen unteren Sammelraum für die Flüssigkeit, einen mittleren Sammelraum für die Gewebezellen und einem oberen Vakuumraum aufteilt, wobei der untere Sammelraum für die Flüssigkeit und der obere Vakuumraum am Sammelraum für die Gewebezellen vorbei miteinander verbunden sind.
Derartige Verfahren und Vorrichtungen werden in der Medizintechnik eingesetzt siehe z.B. US-A-2003/0042187.

Es ist aus vielen Bereichen der Humanmedizin bekannt, Gewebezellen aus einem menschlichen Körper zu entnehmen, um sie beispielsweise zu entsorgen, weil sie überflüssig sind, oder um sie wieder aufzuarbeiten, weil sie aus einem bestimmten Grund wieder benötigt werden.
Dazu werden in herkömmlicher Weise Gewebeteile mechanisch aus der biologischen Struktur herausgetrennt, was sehr schmerzhaft und strapaziös für den Patienten ist. An Stelle der mechanischen Trennung werden auch Ultraschalltechniken zum Lösen von Gewebezellen mit einer anschließenden Absaugung angewendet, die aber in ihrer Wirkung unkontrolliert arbeiten und daher auch sehr belastend für den menschlichen Körper sind.
In der letzten Zeit hat sich das Wasserstrahltrennverfahren verstärkt durchgesetzt, bei dem die zu entnehmenden Gewebezellen in schonender Weise mit Hilfe eines definierten Strahls einer Arbeitsflüssigkeit von den benachbarten Gewebezellen getrennt und die so gelösten Gewebeteile zusammen mit der Arbeitsflüssigkeit und den körpereigenen Flüssigkeiten wieder absaugt werden. Das so abgesaugte Gemisch von Gewebezellen und Flüssigkeit muss anschließend immer dann getrennt werden, wenn die Gewebezellen wieder einer Verwendung zugeführt werden sollen. So werden aus kosmetischen Gründen entnommene und aufbereitete Fettzellen wieder an einer anderen Stelle desselben menschlichen Körpers injiziert. Vitale Gewebezellen aus einer biologischen Struktur, beispielsweise der Leber, werden außerhalb des menschlichen Körpers durch Teilung vermehrt und die so gezüchteten Gewebezellen später dem betreffenden Organ eines Patienten wieder zugeführt.

Zur Trennung der entnommenen Gewebezellen von der Flüssigkeit werden verschiedene Verfahren und Vorrichtungen angewendet.
So sind unter der Bezeichnung "LipiVage" ein Verfahren und eine Vorrichtung der Firma Genesis Biosystems, Inc., Lewisville, Texas 75067 bekannt, die nach dem Prinzip der Druckfilterung arbeiten. Hierbei wird das Gemisch von Gewebezellen und Arbeitsflüssigkeit von einer Kolben-Zylindereinheit aufgesaugt und anschließend unter einem solchen Druck gesetzt, dass die Flüssigkeit aus den Gewebezellen herausgedrückt wird. Die Gewebezellen werden also trocken gepresst, was die Gewebezellen natürlich stark belastet und sie überwiegend unbrauchbar macht.

Zur weitestgehenden Schonung der entnommenen Gewebezellen werden daher die Gewebezellen und die Flüssigkeit häufig durch eine nach den Gesetzen der Schwerkraft ablaufende Sedimentation voneinander getrennt, in dem sich die leichteren Gewebezellen über eine längere Zeit an der Oberfläche der schwereren Flüssigkeit absetzen. Anschließend werden die Gewebezellen zentrifugiert, um auch die noch anhaftenden Restmengen an Flüssigkeit von den Gewebezellen abzutrennen. Dieses Trennverfahren nimmt viel Zeit in Anspruch und benötigt eine zusätzliche Zentrifuge. Das macht das Verfahren und die entsprechende Vorrichtung teuer. Dieses Verfahren ist aber auch nicht stressfrei für die Gewebezellen, weil die Schwerkraftfiltration zu lange dauert und während der nachfolgenden Zentrifugation Zentrifugalkräfte auf die Gewebezellen wirken. Die Gewebezellen nehmen dadurch starken Schaden.

Ebenfalls nach den Gesetzen der Schwerkraft arbeiten ein Verfahren und eine Vorrichtung der Firma Serres Oy, Kurikantie 287, FIN-61850 Kauhajoki as, bei denen die Gewebezellen aus der Flüssigkeit ausgefiltert werden. Dazu ist die entsprechende Vorrichtung im Saugkanal der Wasserstrahltrenneinrichtung eingeordnet, sodass der Innenraum der Vorrichtung unter Vakuum gesetzt ist. Die Vorrichtung besitzt einen zylindrischen Siebbehälter mit einem eingehängten Siebkorb mit unteren und seitlichen Sieböffnungen, die beide im Durchmesser und in der Höhe so aufeinander abgestimmt sind, dass ein unterer Sammelraum und ein umlaufender Ringraum für die Arbeitsflüssigkeit entsteht. Die Zuführung des Gemisches in den Siebkorb erfolgt deckelseitig und die Abführung der reinen Flüssigkeit aus dem unteren Sammelraum des Siebbehälters bodenseitig. Während der Filterung lagern sich die Gewebezellen fortwährend im Siebkorb ab, während der Vakuumstrom über den seitlichen Ringraum an den Gewebezellen vorbei strömt. Zur Entnahme der ausgefilterten Gewebezellen wird der Siebbehälter geöffnet und eine Saugspritze eingesetzt, mit der die Gewebezellen aus der untersten Schicht abgezogen werden.
Auch dieses Verfahren ist sehr zeitaufwendig und verlangt ein nachfolgendes Zentrifugieren. Außerdem werden die Gewebezellen ebenfalls in unzulässiger Weise belastet, weil sie während des ganzen Prozesses der Schwerkraft ausgesetzt sind.

Der Erfindung liegt daher die Aufgabe zu Grunde, ein gattungsgemäßes Verfahren und eine gattungsgemäße Vorrichtung zu entwickeln, bei denen die Schonung der Gewebezellen verbessert und die Filtrationszeit verkürzt wird. Diese Aufgabe wird verfahrensseitig dadurch gelöst, dass der innerhalb des Gewebesammelbehälters vom Gemisch getrennte Vakuumstrom zur Erzeugung eines zusätzlich auf das Gemisch wirkenden Druckes zeitweise unterbrochen wird.

Vorrichtungsseitig wird die Aufgabe dadurch gelöst, dass die Verbindung zwischen dem unteren Sammelraum für die Flüssigkeit und dem oberen Vakuumraum eine absperrbare Bypassleitung ist.
Das neue Verfahren und die neue Vorrichtung beseitigen die genannten Nachteile des Standes der Technik.
Dabei liegt der besondere Vorteil des neuen Verfahrens und der neuen Vorrichtung zunächst darin, dass alle aus dem Vakuum auftretenden Druckkräfte von den Gewebezellen fern gehalten werden. Dadurch trennen sich die Flüssigkeit und die Gewebezellen allein durch die auf das Gemisch wirkende Schwerkraft. Das schont die Vitalität der Gewebezellen. Es besteht jetzt aber auch die Möglichkeit, durch das Absperren der Bypassleitung einen zusätzlichen, aus dem Vakuumstrom entstehenden Druck auf die Gewebezellen auszuüben. Das geschieht vorzugsweise unmittelbar vor der Entnahme der Gewebezellen, sodass die Gewebezellen diesem erhöhten Druck nur kurzzeitig ausgesetzt sind. Mit der Hinzuschaltung des zusätzlichen Druckes werden die Restmengen an Flüssigkeit aus den Gewebezellen gepresst. Das beschleunigt den Filterungsprozess und macht ein nachträgliches Zentrifugieren überflüssig.
Es ist verfahrensmäßig besonders zweckmäßig, wenn die in ihrem Sammelraum befindliche Flüssigkeit so angestaut wird, dass sich ihr Pegel oberhalb der Filterscheibe einstellt. Das führt dazu, dass die unterste Schicht von Gewebezellen ständig Kontakt mit der Flüssigkeit hat. So entwickeln sich aus der Flüssigkeit Auftriebskräfte, die auf die unteren Gewebezellen wirken und der Schwerkraft der Gewebezellen entgegen wirken. Während der reinen Schwerkraftfilterung bleiben die Gewebezellen dadurch fast belastungsfrei, sodass die Gewebezellen in hoher Qualität geerntet werden können. Während der kombinierten Schwerkraft-/Druckfilterung mit abgesperrter Bypassleitung wirken die aus der Flüssigkeit entstehenden Auftriebskräfte der Schwerkraft und den Vakuumkräften entgegen, sodass eine Verfestigung der untersten Schichten der Gewebezellen unmittelbar an der Filterscheibe verhindert wird, womit sich die Durchlassfähigkeit der Filterscheibe verbessert. Dadurch kann die Flüssigkeit leichter abtropfen, ohne dass die Gewebezellen stärker belastet werden.
Zweckmäßige Ausgestaltungen ergeben sich aus den Unteransprüchen 2, 3 und 5 bis 9.

Die Erfindung soll anhand eines Ausführungsbeispieles näher erläutert werden.
Dazu zeigen:
- Fig. 1:: eine schematische Darstellung einer Einrichtung zum Wasserstrahltrennen,
- Fig. 2:: eine Vorrichtung zum Trennen von Gewebeteilen aus einer Flüssigkeit in einer ersten Ausführungsform und
- Fig. 3:: eine Vorrichtung zum Trennen von Gewebeteilen aus einer Flüssigkeit in einer zweiten Ausführungsform.

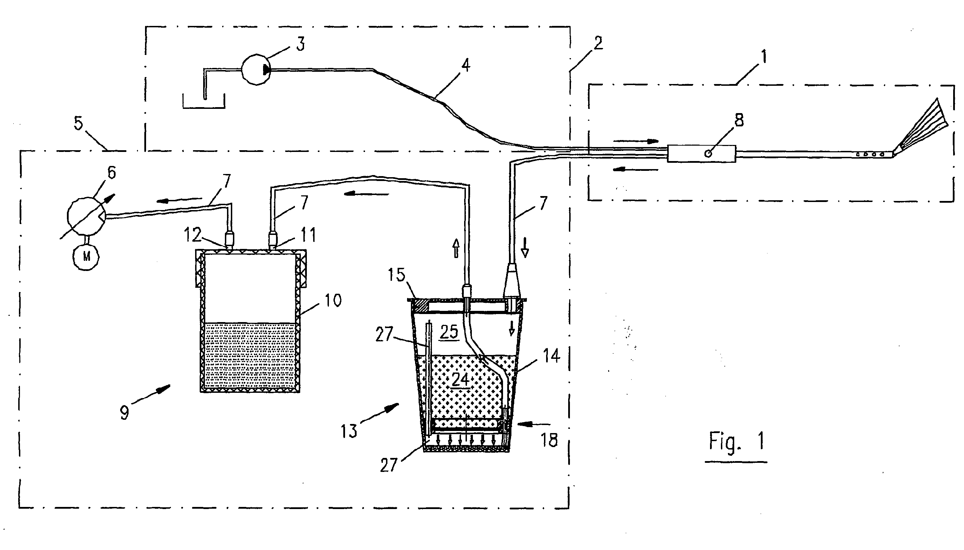

Nach der Fig. 1 besteht die Einrichtung zum Wasserstrahltrennen in herkömmlicher Weise aus einem von der Hand eines Operateurs bedienten Applikator 1 zum wasserstrahlunterstützten Trennen und Absaugen von Gewebezellen aus einer biologischen Struktur, aus einer Druckstrahleinrichtung 2 mit einem Druckerzeuger 3 und einer Druckleitung 4 zur Versorgung des Applikators 1 mit einem definierten Flüssigkeitsstrahls sowie aus einer Saugeinrichtung 5 zur Entsorgung der abgetrennten Gewebeteile und der verwendeten Arbeitsflüssigkeit und der körpereigenen Flüssigkeit aus dem Applikator 1. Zur Saugeinrichtung 5 gehört ein Vakuumerzeuger 6 und eine Saugleitung 7, wobei die Saugleitung 7 den Vakuumerzeuger 6 mit dem Applikator 1 durchgehend verbindet. Im Bereich des Applikators 1 besitzt die Saugleitung 7 einen verschließbaren Bypass 8, der die Saugleitung 7 mit der Atmosphäre verbindet. In der Saugleitung 7 befmden sich zunächst ein Restflüssigkeitssammler 9 für die abgesaugte Flüssigkeit mit einem verschließbaren Auffangbehälter 10 sowie einem Eingangsanschluss 11 und einem Ausgangsanschluss 12 für die Saugleitung 7. Der Restflüssigkeitssammler 9 für die ausgefilterte Flüssigkeit ist in Saugrichtung vor dem Vakuumerzeuger 6 angeordnet. In Saugrichtung vor dem Restflüssigkeitssammler 9 für die Flüssigkeit und in unmittelbarer Nähe zum Applikator 1 befindet sich ein Gewebezellensammler 13.

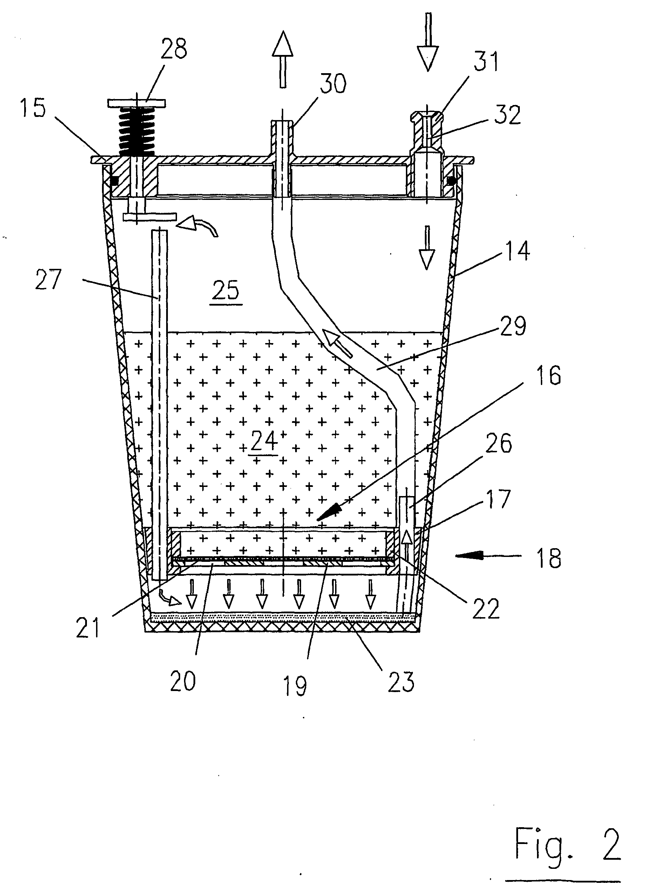

Dieser Gewebezellensammler 13 besteht in seiner ersten Ausführungsform nach der Fig. 2 aus einem zylindrischen Sammelbehälter 14, der mit einem Deckel 15 druckdicht verschlossen ist. Der Sammelbehälter 14 ist vorzugsweise zylindrisch und sich konisch zum Boden des Sammelbehälters 14 verjüngend ausgebildet. Vorzugsweise ist der Sammelbehälter 14 zur Beobachtung des Füllungsstandes und zur Bewertung des Zustandes der aufgefangenen Gewebezellen transparent ausgeführt. Zur Beobachtung des Füllungsstandes besitzt der Sammelbehälter 14 eine Füllstandsanzeige. Im Sammelbehälter 14 befindet sich ein Filtereinsatz 16. Dieser Filtereinsatz 16 besitzt einen Stützring 17, der mit seinem Außendurchmesser so auf den Innendurchmesser des Sammelbehälters 14 abgestimmt ist, dass der Filtereinsatz 16 sich mit einem vorbestimmten Abstand zum Boden des Sammelbehälters 14 an der konischen Wand des Sammelbehälters 14 abstützt und so in eine fixierte Lage kommt. Der Innendurchmesser des Stützrings 17 ist abgestuft ausgebildet, sodass sich eine Auflage für eine Filtereinheit 18 ergibt. Diese Filtereinheit 18 besteht aus einer zu unterst angeordneten und stabilen Stützscheibe 19 mit großformatigen Durchbrüchen 20, einer oder mehreren Filterscheiben 21 mit vorbestimmten Filteröffnungen und einem zu oberst angeordneten Haltering 22. In dieser Anordnung drückt der Haltering 22 die Filterscheibe 21 und die Stützscheibe 19 auf die Auflage der Filtereinheit 18 mit Hilfe seiner Schwerkraft, mit Hilfe von Klemmkräften oder durch einen Formschluss mit dem Stützring 17. Dabei ist die Ausbildung des oberen Halteringes 22 so ausgebildet, dass er sich zum Wechseln der Filterscheibe 21 leicht herausnehmen lässt und dass er unterschiedliche Einbauhöhen realisieren kann, um sich an verschiedene Stärken der einen oder der mehreren Filterscheiben 21 anpassen zu können.

Durch die Anordnung des Filtereinsatzes 16 in der vorbestimmten Höhe ergeben sich unterhalb des Filtereinsatzes 16 ein unterer Sammelraum 23 für die Flüssigkeit und oberhalb des Filtereinsatzes 16 ein Sammelraum 24 für die Gewebezellen. Dazu ist das Fassungsvermögen des Sammelbehälters 14 des Gewebezellensammlers 13 so gewählt, dass sich oberhalb des Sammelraums 24 für die Gewebezellen ein Vakuumraum 25 ausbildet.
Der Stützring 17 des Filtereinsatzes 16 wird von einem vertikal ausgerichteten Saugstutzen 26 und einer gegenüberliegend angeordneten und ebenfalls vertikal ausgerichteten Bypassleitung 27 durchdrungen. Die Bypassleitung 27 endet in einem vorbestimmten Abstand unterhalb des Deckels 15 und verbindet somit den unteren Sammelraum 23 für die Flüssigkeit mit dem oberen Vakuumraum 25 unabhängig vom Füllungsstand des Sammelraumes 24 für die Gewebezellen. Die obere Öffnung der Bypassleitung 27 ist verschließbar ausgeführt. Dazu befindet sich im Deckel 15 des Sammelbehälters 16 ein Verschlusselement 28. Dieses Verschlusselement 28 ist federbelastet und von Hand zu betätigen. In einer baulich einfacheren aber in der Handhabung aufwendigeren Ausführung kann die Bypassleitung 27 nach dem Öffnen des Deckels 15 auch direkt von der Hand des Operateurs verschlossen werden.
Der Saugstutzen 26 mündet unterhalb des Filtereinsatzes 16 und bestimmt so den Pegel der Flüssigkeit im Sammelraum 23. Dadurch ergibt sich zwischen dem Sammelraum 23 für die Flüssigkeit und dem Sammelraum 24 für die Gewebezellen ein Freiraum, der die Flüssigkeit von den Gewebezellen trennt. Nach oben ist der Saugstutzen 26 über eine Saugleitung 29 mit einem Ausgangsstutzen 30 verbunden, der sich im Deckel 15 des Gewebezellensammlers 13 befindet. Die Saugleitung 29 ist vorzugsweise ein Schlauch. Im Deckel 15 des Gewebezellensammlers 13 befindet sich weiterhin ein Eingangsstutzen 31, der im Vakuumraum 25 mündet. Dieser Eingangsstutzen 31 ist mit einer konstanten Strömungsdrossel 32 ausgestattet. Diese Strömungsdrossel 32 ist so dimensioniert, dass einerseits das Eindringen des atmosphärischen Druckes bei einem geöffneten Bypass 8 am Applikator 1 in den Saugbereich der Saugeinrichtung 5 auf den Gewebezellensammler 13 abgeschwächt und andererseits der Durchfluss des Gemisches aus Gewebezellen und Flüssigkeiten nicht über Gebühr behindert wird.

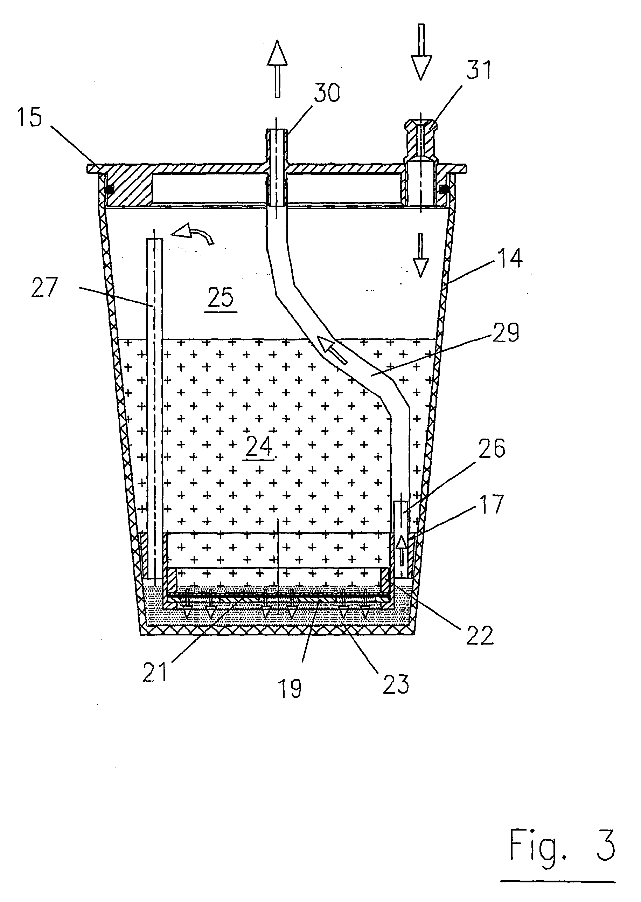

Der Gewebezellensammler 13 in seiner zweiten Ausführungsform gemäß der Fig. 3 unterscheidet sich zur ersten Ausführungsform durch den Wegfall des Verschlusselementes 28 und durch die Ausgestaltung des Filtereinsatzes 16. Dabei ist der Saugstutzen 26 in besonderer Weise derart ausgebildet, dass die untere Mündungsöffnung des Saugstutzens 26 in ihrem Höhenniveau um einen vorbestimmten Betrag über dem Höhenniveau der Filterscheibe 21 liegt, damit sich der Flüssigkeitspegel der im Sammelraum 23 befindlichen Arbeitsflüssigkeit um den vorbestimmten Höhenbetrag über die Filterscheibe 21 erhebt.

Die neue Vorrichtung hat folgende Funktion.
In der Anwendung des Wasserstrahltrennverfahrens tritt aus dem Applikator 1 ein definierter Flüssigkeitstrennstrahl aus, dessen Wirkung durch den in der Druckstrahleinrichtung erzeugte Flüssigkeitsdruck und die konstruktive Ausführung des Applikators 1 bestimmt wird. Diese Wirkung ist darauf gerichtet, Gewebezellen aus einer biologischen Struktur in schonender Weise herauszutrennen. Die so abgetrennten Gewebezellen werden zusammen mit der eingespritzten Arbeitsflüssigkeit und den weiteren körpereigenen Flüssigkeiten durch ein in der Saugeinrichtung 5 erzeugtes Vakuum abgesaugt. Dieses Verfahren wird häufig in der Fettabsaugung angewendet.
Immer dann, wenn die so abgesaugten Gewebezellen einer Wiederverwendung zugeführt werden sollen, werden diese Gewebezellen aus dem GewebezellenFlüssigkeitsgemisch herausgefiltert. Das geschieht durch den Gewebezellensammler 13.

In der Stand-by-Stellung hält der Operateur den verschließbaren Bypass 8 geöffnet, sodass keine Absaugung erfolgt, sondern nur atmosphärische Luft angesaugt und durch den Gewebezellensammler 13 transportiert wird. Dabei passiert die Luft in gedrosselter Form den Eingangsstutzen 31 und gelangt sowohl durch die Bypassleitung 27 als auch durch die frei liegende Filtereinheit 18 in den unteren Sammelraum 23 für die Flüssigkeit und von dort durch die Saugleitung 29 zum Ausgangsstutzen 30.

In der Betriebsstellung ist der verschließbare Bypass 8 durch den Operateur verschlossen, sodass die Saugkraft des Vakuumerzeugers 6 sich auf das Operationsfeld überträgt. Dadurch werden die abgetrennten Gewebeteile und die verschiedenen Flüssigkeiten aufgenommen und zum Gewebezellensammler 13 transportiert.
Im Gewebezellensammler 13 in der ersten Ausführungsform nach der Fig. 2 gelangt das Gemisch von Gewebezellen und Flüssigkeit durch den Eingangsstutzen 31 in den Gewebezellensammler 13 und auf den Filtereinsatz 16. Dabei fällt die Flüssigkeit durch ihre Schwerkraft durch die Filterscheibe 21 und sammelt sich im Sammelraum 23. Die Gewebezellen werden von der Filterscheibe 21 aufgehalten und lagern sich dort ab. Im gleichen Zuge wird der Vakuumstrom durch die Bypassleitung 27 und weitestgehend an den abgelagerten Gewebezellen vorbei in den unteren Sammelraum 23 für die Flüssigkeit geleitet. Hier erfasst der Vakuumstrom die angesammelte Flüssigkeit und transportiert sie durch den Saugstutzen 26 und die Saugleitung 29 zum Ausgangsstutzen 30. Zur Beschleunigung des durch die Schwerkraft erzielten Filtervorganges verschließt der Operateur die Bypassleitung 27 durch das Verschlusselement 28 kurzzeitig. Dadurch wird der hier durchströmende Vakuumstrom abgeschaltet, sodass der Vakuumstrom sich den Weg allein durch die inzwischen angesammelten Gewebezellen sucht. Die dabei auftretenden Kräfte unterstützen die Schwerkraft und pressen die restlichen Flüssigkeitsmengen aus den Gewebezellen. Die so kombinierte Schwerkraft-Druckfiltration beschleunigt den Filtervorgang. Dabei wird die Einwirkungszeit der Vakuumkräfte auf die Gewebezellen kurz gehalten und möglichst unmittelbar vor der Entnahme der Gewebezellen durchgeführt, um die Gewebezellen nicht über Gebühr zu belasten. Anschließend wird die abtransportierte Flüssigkeit im Restflüssigkeitssammler 9 aus dem Vakuumstrom ausgesondert und entsorgt.

Der Gewebezellensammler 13 der zweiten Ausführungsform nach der Fig. 3 arbeitet in gleicher Weise. Allerdings ergibt sich hier der Umstand, dass sich der Pegel der angesammelten Flüssigkeit auf Grund der höher liegenden Mündung des Saugstutzens 26 und der tiefer gelegten Filterscheibe 21 oberhalb der Filterscheibe 21 einstellt, sodass eine untere Schicht der Gewebezellen ständig Kontakt mit der Flüssigkeit hat. Damit wirken die Auftriebskräfte der Flüssigkeit den Schwerkräften der Gewebezellen und den Druckkräften des Vakuumstromes entgegen und entlasten somit die Gewebezellen.

### Liste der Bezugszeichen

- 1: Applikator
- 2: Druckstrahleinrichtung
- 3: Druckerzeuger
- 4: Druckleitung
- 5: Saugeinrichtung
- 6: Vakuumerzeuger
- 7: Saugleitung
- 8: verschließbarer Bypass
- 9: Restflüssigkeitssammler
- 10: Auffangbehälter
- 11: Eingangsanschluss
- 12: Ausgangsanschluss
- 13: Gewebezellensammler
- 14: Sammelbehälter für die Gewebezellen
- 15: Deckel
- 16: Filtereinsatz
- 17: Stützring
- 18: Filtereinheit
- 19: Stützscheibe
- 20: Durchbruch
- 21: Filterscheibe
- 22: Haltering
- 23: Sammelraum für die Flüssigkeit
- 24: Sammelraum für die Gewebezellen
- 25: Vakuumraum
- 26: Saugstutzen
- 27: Bypassleitung
- 28: Verschlusselement
- 29: Saugleitung
- 30: Ausgangsstutzen
- 31: Eingangsstutzen
- 32: konstante Strömungsdrossel

## Patentansprüche

1. Verfahren zum Trennen von Gewebezellen aus einer Flüssigkeit, bei dem ein Gemisch aus den Gewebezellen und der Flüssigkeit in einen Vakuumstrom eingebunden und in einem Gewebesammelbehälter (13) wieder vom Vakuumstrom getrennt wird, wobei die Gewebezellen aus dem Gemisch durch die Schwerkraft ausgefiltert und die verbleibende Flüssigkeit wieder in den Vakuumstrom eingebunden wird,
**dadurch gekennzeichnet, dass** der innerhalb des Gewebesammelbehälters (13) vom Gemisch getrennte Vakuumstrom zur Erzeugung eines zusätzlich auf das Gemisch wirkenden Druckes zeitweise unterbrochen wird.

2. Verfahren nach Anspruch 1,
**dadurch gekennzeichnet, dass** die Unterbrechung des vom Gemisch getrennten Vakuumstromes unmittelbar vor der Entnahme der Gewebezellen erfolgt.

3. Verfahren nach Anspruch 1,
**dadurch gekennzeichnet, dass** die aus der Filterung angesammelte Flüssigkeit so angestaut wird, dass der Pegel der Flüssigkeit sich über dem Höhenniveau der Filterscheibe (21) einstellt.

4. Vorrichtung zum Trennen von Gewebezellen aus einer Flüssigkeit, bestehend aus einem unter Vakuum stehenden Gewebezellensammler (13) mit einer Filtereinheit (18), die den Sammelbehälter (14) in einen unteren Sammelraum (23) für die Flüssigkeit, einen mittleren Sammelraum (24) für die Gewebezellen und einem oberen Vakuumraum (25) aufteilt, wobei der untere Sammelraum (23) für die Flüssigkeit und der obere Vakuumraum (25) am Sammelraum (24) für die Gewebezellen vorbei miteinander verbunden sind,
**dadurch gekennzeichnet, dass** die Verbindung zwischen dem unteren Sammelraum (23) für die Flüssigkeit und dem oberen Vakuumraum (25) eine absperrbare Bypassleitung (27) ist.

5. Vorrichtung nach Anspruch 4,
**dadurch gekennzeichnet, dass** die Bypassleitung (27) durch ein Verschlusselement (28) absperrbar ist.

6. Vorrichtung nach Anspruch 4,
**dadurch gekennzeichnet, dass** der Sammelraum (24) für die Gewebezellen aus dem Sammelbehälter (14) und einer horizontal angeordneten Filtereinheit (18) gebildet wird.

7. Vorrichtung nach Anspruch 6,
**dadurch gekennzeichnet, dass** der Sammelraum (24) für die Gewebezellen von einer vom Sammelraum (23) für die Flüssigkeit zum Ausgangsanschluss (30) führenden Verbindungsleitung (29) durchdrungen ist, wobei der Ausgangsanschluss (30) im Deckel (15) des Gewebezellensammlers (13) angeordnet ist.

8. Vorrichtung nach Anspruch 6,
**dadurch gekennzeichnet, dass** die Filtereinheit (18) aus einer Filterscheibe (21) besteht, die von einem Stützring (17) und einer Stützscheibe (19) getragen und von einem Haltering (22) fixiert wird.

9. Vorrichtung nach Anspruch 8,
**dadurch gekennzeichnet, dass** die Filterscheibe (21) in der Höhe unterhalb der Mündungsöffnung des Saugstutzens (26) für die Saugleitung (29) angeordnet ist.

## Claims

1. A method for separating tissue cells from a fluid, where a mixture of the tissue cells and the fluid is incorporated into a vacuum stream and separated again from the vacuum stream in a tissue collection container (13), wherein the tissue cells are filtered out of the mixture by way of gravity and the remaining fluid is reintegrated into the vacuum stream,
**characterized in that** the vacuum stream separated from the mixture inside the tissue collection container (13) is interrupted temporarily to generate a pressure which acts additionally on the mixture.

2. The method according to claim 1,
**characterized in that** the interruption of the vacuum stream separated from the mixture occurs immediately prior to the removal of the tissue cells.

3. The method according to claim 1,
**characterized in that** the fluid collected from the filtration process is accumulated such that the fluid level adjusts above the height level of the filter disk (21).

4. A device for separating tissue cells from a fluid, consisting of a tissue cell collector (13) under vacuum having a filter unit (18) dividing the collection container (14) into a lower collection space (23) for the fluid, a central collection space (24) for the tissue cells and an upper vacuum space (25),
wherein the lower collection space (23) for the fluid and the upper vacuum space (25) are connected to one another bypassing the collection space (24) for the tissue cells,
**characterized in that** the connection between the lower collection space (23) for the fluid and the upper vacuum space (25) is a bypass line (27) which can be blocked.

5. The device according to claim 4,
**characterized in that** the bypass line (27) can be blocked by a shutoff element (28).

6. The device according to claim 4,
**characterized in that** the collection space (24) for the tissue cells is formed by the collection container (14) and a horizontally arranged filter unit (18).

7. The device according to claim 6,
**characterized in that** the collection space (24) for the tissue cells is penetrated by a connection line (29) extending from the collection space (23) for the fluid to the outlet port (30), wherein the outlet port (30) is arranged in the lid (15) of the tissue cell collector (13).

8. The device according to claim 6,
**characterized in that** the filter unit (18) consists of a filter disk (21) which is supported by a supporting ring (17) and a supporting disk (19) and held in place by a retaining ring (22).

9. The device according to claim 8,
**characterized in that** the filter disk (21) is arranged in height below the mouth opening of the suction port (26) for the suction line (29).

## Revendications

1. Procédé permettant d'isoler des cellules tissulaires contenues dans un liquide, dans lequel un mélange constitué des cellules tissulaires et du liquide est introduit dans un flux sous vide et retiré du flux sous vide dans un réservoir collecteur de tissus (13), les cellules tissulaires étant extraites du mélange par filtration sous l'action de la gravité et le liquide restant étant réintroduit dans le flux sous vide,
**caractérisé en ce que** le flux sous vide isolé du mélange à l'intérieur du réservoir collecteur de tissus (13) est interrompu par intermittence pour la production d'une pression agissant de façon supplémentaire sur le mélange.

2. Procédé selon la revendication 1,
**caractérisé en ce que** l'interruption du flux sous vide isolé du mélange s'effectue tout de suite avant le prélèvement des cellules tissulaires.

3. Procédé selon la revendication 1,
**caractérisé en ce que** le liquide collecté à partir de la filtration s'accumule de telle sorte que le niveau du liquide se règle au-dessus du niveau de hauteur du disque filtrant (21).

4. Dispositif permettant l'isolation de cellules tissulaires contenues dans un liquide, composé d'un réservoir collecteur de tissus (13) sous vide avec une unité de filtration (18) qui divise le récipient collecteur (14) en un espace collecteur inférieur (23) pour le liquide, un espace collecteur médian (24) pour les cellules tissulaires et un espace sous vide supérieur (25), l'espace collecteur inférieur (23) pour le liquide et l'espace sous vide supérieur (25) pour les cellules tissulaires étant raccordés l'un à l'autre le long de l'espace collecteur (24) pour les cellules tissulaires,
**caractérisé en ce que** le raccordement entre l'espace collecteur inférieur (23) pour le liquide et l'espace sous vide supérieur (25) est une conduite de dérivation (27) pouvant être obturée.

5. Dispositif selon la revendication 4,
**caractérisé en ce que** la conduite de dérivation (27) peut être obturée par un élément de fermeture (28).

6. Dispositif selon la revendication 4,
**caractérisé en ce que** l'espace collecteur (24) pour les cellules tissulaires est formé du récipient collecteur (14) et d'une unité de filtration (18) disposée horizontalement.

7. Dispositif selon la revendication 6,
**caractérisé en ce que** l'espace collecteur (24) pour les cellules tissulaires est traversé par une conduite de raccordement (29) conduisant de l'espace collecteur inférieur (23) pour le liquide au raccord de sortie (30), le raccord de sortie (30) étant disposé dans le couvercle (15) du collecteur de cellules tissulaires (13).

8. Dispositif selon la revendication 6,
**caractérisé en ce que** l'unité de filtration (18) se compose d'un disque filtrant (21) qui est supporté par une bague de support (17) et un disque de support (19) et qui est fixé par une bague de retenue (22).

9. Dispositif selon la revendication 8,
**caractérisé en ce que** le disque filtrant (21) est disposé en hauteur au-dessous de l'ouverture d'embouchure de la tubulure d'aspiration (26) de la conduite d'aspiration (29).
